# EUROPEAN PATENT APPLICATION

(11) **EP 3 705 467 A1**
(43) Date of publication of application: **09.09.2020**
(21) Application number: 19161450.2
(22) Date of filing: 07.03.2019
(51) Int. Cl.: C07C 17/10, C07C 19/03

(54) **PROCESS FOR THE DIRECT HALOGENATION OF AN ALIPHATIC HYDROCARBON TO A HALOGENATED ALIPHATIC HYDROCARBON**

(71) Applicant: Studiengesellschaft Kohle MbH, 45470 Mülheim an der Ruhr (DE)
(72) Inventor: SCHUETH, Ferdi, 45470 Mülheim an der Ruhr (DE); LOSCH, Pit, 45470 Mülheim an der Ruhr (DE); BILKE, Marius, 47829 Krefeld (DE); VOZNIUK, Olena, 45470 Mülheim an der Ruhr (DE); BODACH, Alexander, 45468 Mülheim an der Ruhr (DE)

(57) **Abstract**

The present application refers to a process for the direct and selective halogenation of an aliphatic hydrocarbon to a halogenated aliphatic hydrocarbon by a mechanochemical reaction.

## Description

The present application refers to a process for the direct halogenation of an aliphatic hydrocarbon to a halogenated aliphatic hydrocarbon, in particular to a monohalogenation of a lower alkane having 1 to 8 carbon atoms such as methane to a monohalomethane, such as chloromethane, by a mechanochemical reaction.

In the state of art, several processes for converting alkanes into haloalkanes, in particular methane (CH₄) into chloromethane (CH₃Cl) are known. These processes often suffer from the disadvantages encompassing the necessity of corrosive reagents that can be dangerous to store, transport and handle, but also low selectivity to monochloromethane. These processes typically yield a product mixture requiring subsequent separation.

Chloromethane (CH₃Cl), being isostructural with methanol, is an attractive platform molecule that can easily be transformed into a wide variety of chemicals and fuels. CH₃Cl finds application as a methylating agent for polymers and, more importantly, similar to methanol, CH₃Cl can be directly used as a feedstock to produce olefins with zeotype catalysts. A decisive limitation for large scale application of a process converting natural gas into olefins via chloromethane is the low selectivity to monohalogenated products. Low selectivities towards monochloromethane are observed, since CH₃Cl is more readily chlorinated than methane itself. Therefore current industrial processes for synthesizing monochloromethane rely on product separation after a radical reaction, or methanol hydrochlorination. The latter is catalyzed by alumina with a very high selectivity to monochloromethane, but it does not allow the direct upgrade of natural gas and requires the non-trivial production of methanol as an intermediate product.

The high corrosivity and toxicity of elemental chlorine or bromine triggered the development of oxy-halogenation processes. This elegant approach relies on the *in situ* generation of gaseous halogen by feeding a gas stream of the corresponding hydrogen halide together with O₂, which then halogenate alkanes via radical pathways. However, it still requires harsh conditions (T > 400 °C).

At present, no selective process for the direct chlorination of methane to chloromethane has been established.

Surprisingly, the inventors found, that a solid particulate halogenating agent such as trichloroisocyanuric acid (TCCA), N-chlorosuccinimide (NCS), tribromoisocyanuric acid (TBCA) or N-bromosuccinimide (NBS) can be used as safe halogenating agents that are obtainable at large scale and thus readily available at reasonable cost in the inventive process.

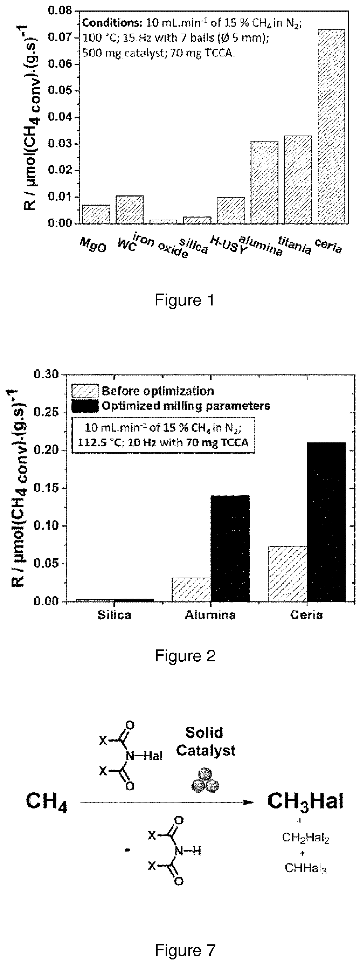

For example, TCCA is a common bleaching and disinfecting agent, *e.g.* for swimming pools. It possesses an exceptionally high active chlorine content (91.5 %), comparable to that of molecular Cl₂ (100 %). Dry TCCA itself is non-corrosive and stable under ambient conditions, and it thermally decomposes only above 230 °C. The most challenging C-H activation, namely the transformation of CH₄ involves the critical step of C-H bond scission to form methyl radicals or methyl-metal intermediates, which would readily react with solvent molecules. Therefore, the inventors developed a gas-solid mechanochemically driven monohalogenation of methane characterized by the advantages that the mechanochemical activation enables the use of a cheap and safe chlorinating agent, abundant catalytic material and overall benign reaction conditions as exemplarily shown in **Figure 7****,** illustrating the investigated mechanochemical monohalogenation of methane at low temperatures (T < 150 °C).

Thus, the present invention refers to a process for reacting an aliphatic hydrocarbon, preferably an alkane having 1 to 8 carbon atoms, in particular methane, with a solid halogen source in a reactor wherein mechanical forces are applied to the reaction system. The application of mechanical forces to the reaction mixture in the reaction system, in the reactor, can be achieved by milling the reaction mixture in a milling jar in the presence of milling balls and a particulate inert solid milling medium or by subjecting the reaction mixture to an adapted rotating tubular furnace or an adapted circulating fluidized bed (CFB) treatment in a fluidized bed reactor equipped with a cyclone separator. According to the invention, the application of mechanical forces can be controlled depending on the reaction partners and the other reaction conditions (**Figures 1 - 3**).

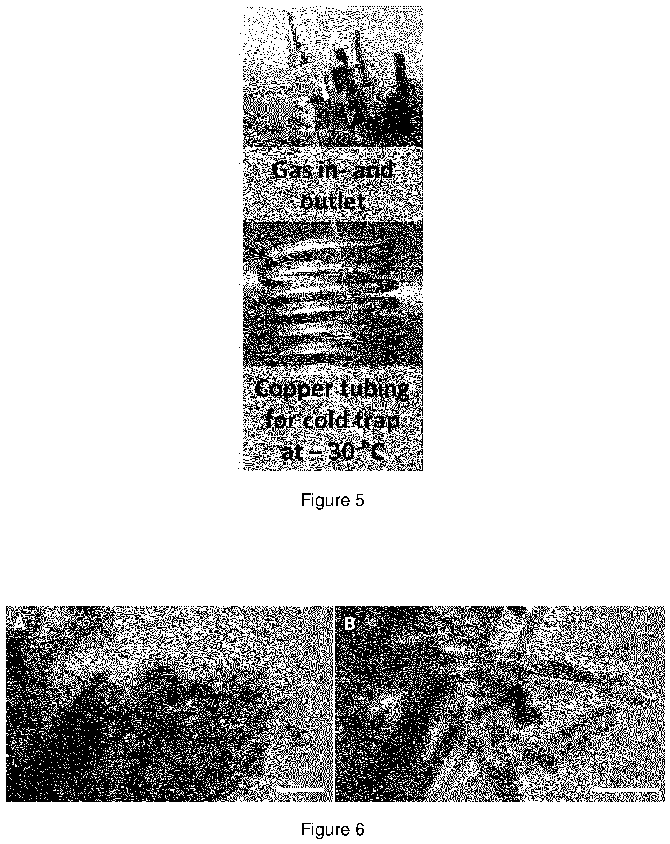

Generally, the inventive process is characterized by reacting an aliphatic hydrocarbon having 1 to 8, preferably 1 to 6 carbon atoms, more preferably 1 to 3 carbon atoms, in its gaseous state with a solid particulate halogen source in the presence of a particulate inert milling medium in a reactor, optionally in the presence of milling balls, at a temperature below the decomposition temperature of the said solid particulate halogen source, whereby the reactor is equipped with at least one inlet and at least one outlet and the alkane is led into the reactor preferably at a continuous flow rate while the resulting reaction mixture is led out of the reactor. The resulting reaction mixture comprising unreacted reaction partners and the reaction product is then separated into its components and the obtained unreacted aliphatic hydrocarbon, and optionally unreacted gaseous halogen, if used in the inventive process, can be recycled to the inlet of the reactor (**Figure 5**).

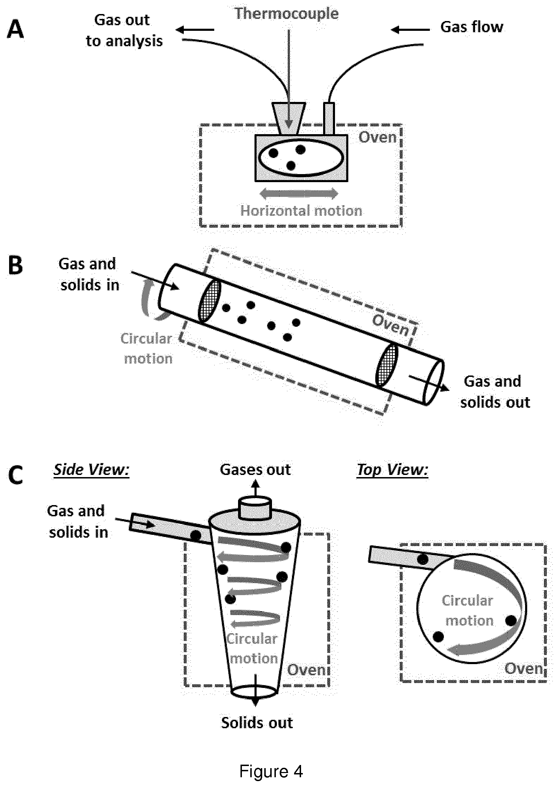

Depending on the scale of application that is aimed, the reactor can be a container such as a ball mill with a milling jar and milling balls (small scale), or a rotating tubular furnace equipped with balls and sieves (medium scale) or a fluidized bed reactor with a cyclone separator unit allowing for an efficient transfer of kinetic energy (large scale) and is generally equipped with at least one inlet for the gaseous aliphatic hydrocarbon having 1 to 8, preferably 1 to 6 carbon atoms, more preferably 1 to 3 carbon atoms (**Figure 4**).

In one embodiment of the inventive process, the solid particulate halogen source is selected from halogen containing electrophilic molecules, preferably nitrogen-halogen bond containing molecules being preferably selected from trichloroisocyanuric acid, tribromoisocyanuric acid, N-chlorosuccinimide or N-bromosuccinimide. If desired, the process can optionally be carried out in the presence of a halogen in gas form which is preferably the same halogen as used in the solid particulate halogen source.

In one embodiment, the inventive process is characterized by reacting methane with trihaloisocyanuric acid such as tribromo- or trichloro-isocyanuric (TBCA or TCCA) as particulate halogen source in the presence of a particulate milling medium, also referred to as solid catalyst in a reactor such as a ball mill with a milling jar and balls at a temperature below the decomposition temperature of trihaloisocyanuric acid, whereby the milling jar is equipped with a gas inlet and a gas outlet and the methane is led into the milling jar preferably at a continuous flow rate and the resulting gas mixture is led out of the jar.

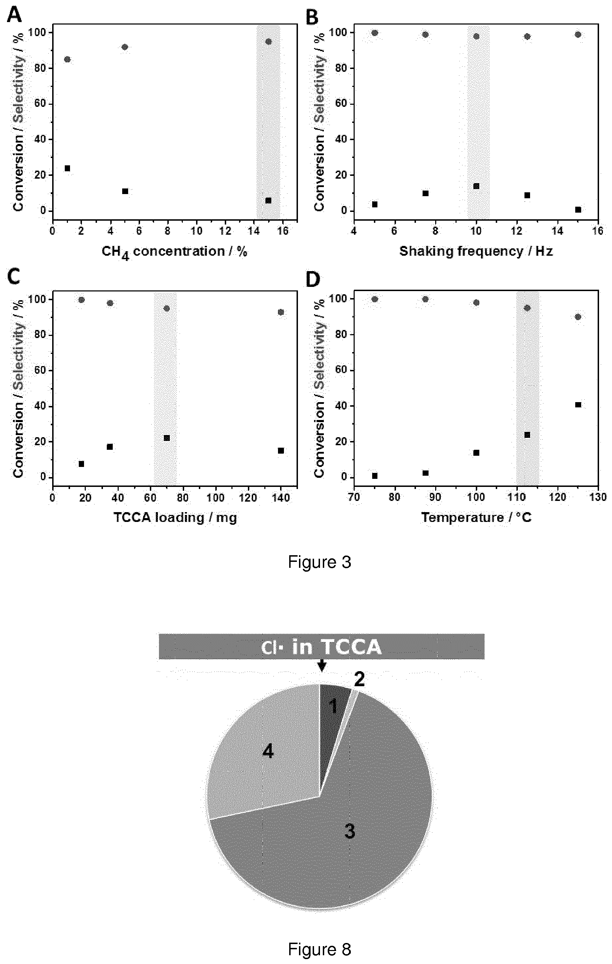

The inventors have found out that the reaction conditions have to be chosen according to the specific conditions of the reactor such as a circulating fluidized bed reactor (CFB) or a ball mill, preferably a shaker ball mill, the milling jar, the milling balls, the particulate milling medium, the ratio of the reaction partners methane and solid halogen source such as TBCA or TCCA and the reaction temperature which is applied to the reactor as such as milling jar (**Figure 2** and **Figure 3**).

Generally, the aliphatic hydrocarbon having 1 to 8, preferably 1 to 6 carbon atoms, more preferably 1 to 3 carbon atoms, such as methane, ethane, propane, butane, pentane, hexane, heptane, octane and their isomers, is led in its gaseous state into the reactor, such as a milling jar, preferably at a continuous flow rate and preferably diluted with an inert gas such as N₂ to a concentration between up to 20 vol.-% of the above mentioned aliphatic hydrocarbon. Depending on the boiling point of the aliphatic hydrocarbon under normal pressure (1 atm), it may be necessary to reduce the pressure inside of the reactor in order to vaporize the aliphatic hydrocarbon to gas state and to react it with the solid particulate halogen source in the presence of a particulate inert milling medium.

The reaction temperature is kept below the decomposition temperature of the solid particulate halogen source, such as trihaloisocyanuric acid, preferably tribromo-or trichloro-isocyanuric acid, preferably in the range of 75°C to 150°C, more preferably depending on the solid catalysts used between 100°C and 130°C (**Figure 3**).

The reactor such as a milling jar is generally shaken at frequency of 7 to 15 Hz, preferably 9 to 11 Hz, depending on the loading conditions (**Figure 3**). The walls of the reactor are generally made of or at least partially covered with steel, WC, PTFE, Agate or ZrO₂.

In the inventive process, the used milling balls are balls generally made of steel, WC, Agate, ZrO₂, coated by PTFE or combinations thereof, said balls preferably having a diameter between 0.1 to 10 cm. The size of the balls is depending on the use at different scales and the balls with mixed diameters can be used.

The particulate milling medium is selected from an inorganic oxidic Lewis acidic material, preferably oxides of transition metals, of rare earth elements and of group thirteen elements, such as titania, zirconia, alumina, in particular γ-alumina, ceria and/or mixtures thereof (**Figure 1**). Generally, the use of ceria or alumina is preferred. The particle size of the particulate milling medium is generally in a size range of below 500nm, preferably below 100nm and more preferably below 50nm in order to increase the specific surface area (**Figure 6**).

The particulate milling medium is used in an amount in the reactor so that the solid halogen source such as TBCA or TCCA is finely deposited on the surface of the particles of the solid particulate milling medium under the action of the milling balls. Generally, the mass ratio between the particulate milling medium and halogenating agent is between 1 and 100, and preferably between 3 and 15.

The invention is further illustrated by the following Figures and Experiments. The Figures show:
- **Figure 1:**: Reaction rate (R) for different materials under identical milling conditions; reactions were carried out over 40 min. In all the reactions only CH₃Cl was detected;
- **Figure 2:**: Reaction rate averaged over a reaction over 40 min for alumina, ceria and silica before and after optimization of milling parameters (optimized parameters in box);
- **Figure 3:**: Optimization of various parameters for the milling reaction. Average conversions of methane are given in black squares and average selectivity towards CH₃Cl in red circles over a run of 40 min.
A) Different methane concentrations (Conditions: 10 mL.min⁻¹ of x % CH₄ in N₂; 112.5 °C; 10 Hz with 7 balls (Ø = 5 mm); 500 mg Al₂O₃; 70 mg TCCA);
B) Various shaking frequencies (Conditions: 10 mL.min⁻¹ of 15 % CH₄ in N₂; 100 °C; x Hz with 7 balls (Ø = 5 mm); 500 mg Al₂O₃; 70 mg TCCA);
C) Increasing TCCA loading (Conditions: 10 mL.min⁻¹ of 15 % CH₄ in N₂; 112.5 °C; 10 Hz with 7 balls (Ø = 5 mm); 500 mg Al₂O₃; x mg TCCA) and
D) Increasing temperatures (Conditions: 10 mL.min⁻¹ of 15 % CH₄ in N₂; x °C; 10 Hz with 7 balls (Ø = 5 mm); 500 mg Al₂O₃; 70 mg TCCA) were studied. Highlighted conditions are the selected optimal reaction conditions for this study, red squares were deliberately chosen while the blue conditions were chosen based on an optimal behaviour as a function of the studied parameter;
- **Figure 4:**: A gas flow ball milling experimental setup in three embodiments for application at different scales;
A) shows a ball mill with a milling jar and milling balls equipped with gas inlet and outlet allowing for a continuous gas feed for small scale application,
B) shows a rotating tubular furnace equipped with balls and sieves allowing a continuous feed of both solids and gases at a medium scale,
C) shows a fluidized bed reactor with balls and a cyclone separator unit allowing for an efficient transfer of kinetic energy (large scale).
- **Figure 5:**: A metallic (copper) cold trap to be inserted in a liquid cooling bath at-30 °C permitting ready product separation and gas stream recycling;
- **Figure 6:**: Transmission electron micrographs of two tested and active ceria samples. A) Nano polyhedra, B) Nano rods, scale bar in both images 50nm;
- **Figure 7:**: Schematic of the studied mechanochemical reaction. The selective monohalogenation of methane by solid chlorinating agents on solid catalysts with mechanical activation, Hal = Cl or Br and X = any substituent composed of N, C, H atoms;
- **Figure 8:**: A closed chlorine balance was ascertained: **(1)** represents the share of Cl lost on the solid surface; **(2)** HCI; **(3)** recombined Cl₂ which can be recycled; **(4)** selectively formed CH₃Cl;

### Experimental Section

Initial tests were carried out in a Fritsch planetary ball milling setup (Pulverisette 6) under methane pressure at room temperature (20°C). These tests gave minor conversion of methane with acidic milling medium. Observed trace products (CH₃Cl and CH₂Cl₂) were identified by mass spectrometry.

Further experiments were performed in a Retsch shaker mill equipped with either a tungsten carbide (WC) milling jar or a steel jar modified for continuous gas flow reactions, described in the prior art. Effluent gases were analyzed by means of a GC-FID.

The inventors investigated a set of abundant solid materials in this milling setup with different physico-chemical properties: basic MgO was compared to redox active Fe₂O₃, weakly acidic SiO₂, Brønsted acidic zeolite H-USY (Si/AI = 6), non-reducible (y-Al₂O₃), and reducible Lewis acids (ZrO₂, TiO₂ and CeO₂), and to the milling jar material WC. **Figure 1** shows the respective reaction rates in µmol(CH_{4 conv}).(g.s)⁻¹ averaged over a reaction of 40 minutes. The Lewis acidic materials result as the most promising for the given transformation.

The benefit of the setup of the inventors is the possibility to screen catalytically relevant parameters in a systematic manner. Temperature (from RT to 150 °C), contact time (i.e. composition and rate of gas flow) and the transfer of kinetic energy or momentum (by varying the shaking frequency and number / mass / material of balls) were studied. Thus milling parameters could be thoroughly optimized (**Figure 3**). As a result of this optimization the loading of the chlorinating agent as well as the shaking frequency emerged as key parameters. **Figure 2** displays reaction rates for ceria, alumina and silica before and after the optimization. The reaction rates for ceria and alumina were increased by a factor of 3 - 4. In contrast silica still showed no detectable activity.

It is worthy to mention that TCCA is synthesized from cyanuric acid, Cl₂ and NaOH at large scale, thus it is imaginable to reactivate cyanuric acid and to reuse Cl₂ after the reaction. The inventors have shown that a simple washing step with water efficiently removes residual cyanuric acid. In addition, N-chlorosuccinimide (NCS) has been tested as an alternative chlorinating agent (for both same mass and same molar amount), it was concluded that it was less efficient comparing the yields for reactions loaded with 905 µmol of active chlorine: 28 µmol(CH₃Cl) for NCS and 186 µmol(CH₃Cl) for TCCA. Also gaseous Cl₂ was tested as a chlorine source with and without the addition of TCCA. These tests revealed that the chlorination of methane by Cl₂ is not affected at all by milling, while the combined use of dilute Cl₂ and TCCA also worked as an efficient chlorine source for the selective monochlorination. The reaction with only Cl₂ produced ca. 10 µmol(CH₃Cl) during one reaction, whereas the reaction with both TCCA and a diluted Cl₂ flow gave the highest yield with ca. 1000 µmol(CH₃Cl).

Since TCCA is a solid crystalline chlorinating agent, its structure and solid state properties may also be related to its reactivity. Therefore, samples were analysed by XRPD data of a quality that allowed structure solution and Rietveld refinement. It can - unsurprisingly - be seen that the molecular structure is flat and confirms the extension of the sp² hybridization throughout the whole molecule. Therefore, TCCA can be considered as a Hückel aromatic molecule with 18e⁻ in the extended conjugated π-etectron-system. These molecules interact via electrostatic and π-stacking supramolecular forces to form a hexagonal layered material. Layered materials such as graphite and h-BN can be considered as relatively soft. Hence, a harder milling medium is necessary to molecularly disperse TCCA on the oxide surface, but also to assure an efficient energy transfer upon mechanochemical activation. The textural properties of the metal oxides remained unaffected by the moderate mechanical impact during reaction with 7 balls and a total mass of 7.16 g, shaken at a frequency of 10 Hz.

If a larger scale application of the studied process is envisaged, a continuous feed of solids in addition to the continuous gas flow could be a pathway to solve the current technical limitation. From an engineering point of view, circulating fluidized beds with cyclone units will be key to circumventing such limitations. In a fluidized bed, frequent collisions between particles themselves but also between walls and particles lead to fouling. These collisions, however, make them also interesting as potential large scale gas-solid mechanochemical continuous setups. Especially when considering the moderate mechanical impact required to trigger this reaction, one could think of co-feeding solids and small balls to a fluidized bed, or to use a modified rotating tube furnace equipped with sieves to retain the balls, for a large scale haloalkane production, such as a chloromethane production.

### Experiments

### 1.1. Materials

All chemicals and gases used herein were purchased from commercial suppliers and used as received, unless stated differently. Purities are given in brackets: Trichloroisocyanuric acid (> 95%), cyanuric acid (98%), cerium(III)nitrate hexahydrate (99.99%), γ-aluminum oxide (catalyst support, 200 m²/g), quartz sand (>99.995%), titanium oxide (>99.98%, rutile), iron(III)oxide (< 96%), ZrO₂ (> 98 %) magnesium oxide (> 99%), tungsten carbide (99.5%), H-USY (surface area 730 m² g⁻¹, 6:1 Si/AI, calcined at 550°C), d₆-DMSO (99.5%), CD₃CN (99.6%). The gas mixture consisting of 0.4% CH₄, 4% O₂ and 95.6% Ar was prepared in house.

CeO₂ nano-polyhedra were synthesized according to a literature procedure. In brief, Ce(NO₃)₃·6H₂O was calcined in air at 350°C (2°C min⁻¹) for 2 h, producing a yellow powder. The powder was then ground in a mortar and particles with a size in the range of 5-20nm were collected.

### 1.2. Experimental setup for the monohalogenation reaction

### 1.2.1 Gas flow milling setup, reactions and analyses

Continuous catalytic experiments were carried out inside a modified 25 mL tungsten carbide milling vial, which was attached to a Retsch MM400 shaker mill. In a typical experiment, the milling vial contained the solid milling medium (500 mg), the solid chlorinating agent TCCA (70 mg) and seven tungsten carbide balls (d = 5 mm; m(ball) = 1,02 g). The jar is equipped with a gas in- and outlet to enable a continuous flow of reactant gas (0.5 - 15 vol.% CH₄ in N₂). During a typical reaction, the vial was shaken at a frequency of 10 Hz, while the whole milling jar was set to a temperature of 112.5°C. Experimental setup is depicted in **Figure 4A****.** Stainless steel was also used as an alternative material for the milling jar and balls.

Mass spectrometry analysis of the effluent gases was carried out with a Thermostar mass spectrometer from Balzers Instruments in the range of 1 - 100 m.z⁻¹.

Continuous sampling of the outlet gas flow was possible thanks to a direct connection via a pneumatic six way valve to a GC (Agilent 6850) equipped with apolar column (DB-1, 30 m, 0,25 mm, 0,25 µm) and a flame ionization detector (FID). The presence of monochloromethane was confirmed by MS and response factors on the GC were determined by using pure products; CH₄, CH₃Cl, CH₂Cl₂ and CHCl₃. The herein used column permitted the separation of the four mentioned molecules. For separation, the following conditions were applied: Tₒᵥₑₙ = 40 °C; Vₗₒₒₚ = 300 µL; Split 130:1; flow carrier gas (N₂) 1.5 mL.min⁻¹.

HCl-formation test was performed on the setup described above, with the following reaction conditions, a flow of 10 mL.min⁻¹ of CH₄ (15 %), 10 Hz shaking frequency, 7 balls, 500 mg CeO₂ and 70 mg TCCA at 112.5 °C. Instead of analyzing the reaction flow with online GC the gas was washed with a saturator containing 10 mL dist. H₂O (Figure 4). The pH of this solution was analyzed before and after reaction by means of a Titrino Plus 848 pH meter from Metrohm. In such a reaction 70 mg of TCCA correspond to 905 µmol of active chlorine in the reaction. After a full reaction of 40 min shaking, The inventors previously produced 270 µmol of CH₃Cl. The inventors now observed a decrease in the pH from 5.3 to 3.1. This result can be interpreted as follows:

| | |
|---|---|
| with | c(H⁺) = C(HCl_{prod.}) |
| The inventors can write | c(HCl_{prod} ) = 10^{-3.1} -10^{-5.3} mol.L⁻¹ = 7.9. 10⁻⁴ mol.L⁻¹ |
| considering V = 10 mL | n(HCl_{prod}.).(n(Cl_{active}))⁻¹ = 7.9 µmol / 905 µmol = 0.87 % |

The inventors can conclude that less than 1 % of the active chlorine in TCCA has formed HCI, while the inventors know from their catalytic data that more than 30 % of active chlorine formed CH₃Cl.

Cl₂ quantification was carried out on the same setup as described for HCI titration, applying an off-line iodometric titration of polyiodides here simplified as triiodide, which form upon bubbling the reaction gas stream containing through 10 mL of an aqueous KI solution (0.2 M). The formation of triiodide species could be optically ascertained as the clear solution gradually turned orange, yet only after switching on the milling. The formed triiodide was then titrated with a standard sodium thiosulfate (0.1 M) solution (Fisher Scientific, for volumetric analysis).
For the idodometric titration the following reactions were considered:
(1)

   Cl₂ + 2 I⁻→ I₂ + 2 Cl⁻
(2)

   I₂ + 2 I⁻ ⇄ I₃⁻
(3)

   I₃⁻ + 2 S₂O₃²⁻ → 3 I⁻ + S₄O₆²⁻
From these it was drawn that: n(I₂) = n(Cl_{2,prod.}) = 2 n(Cl_{active lost})
Hence n(S₂O₃²⁻) = n(Cl_{active lost})
Therefore: n(S₂O₃²⁻) = 6.4 ± 0.4 mL.0.1 mol.L⁻¹ = 640 ± 40 µmol = n(Cl_{active lost}) n(Cl^{•}_{,prod.}).(n(Cl_{active}))⁻¹ = 640 ± 40 µmol / 905 µmol ≈ 70 ± 5 %
A considerable share of the loaded active Cl is thus swept off during the reaction as Cl₂, indicating that the recombination reaction between two Cl· radicals occurs faster than the C-H bond scission, and consecutive CH₃Cl formation by recombination of CH₃· and Cl·. Nonetheless dilute Cl₂ can be fed in a cyclic manner over the catalyst as it was shown that dilute Cl₂ in combination with TCCA is an efficient chlorination source.
Finally, surface elemental analysis was performed by XPS and resulted in a Cl / N ratio on the solid milled surface of < 1 / 18 (1 / 1 before reaction). Hence, only approx. < 5 % of the initially loaded active Cl remained in the final solid, either by forming strong bonds with the support, or due to incomplete decomposition of TCCA.

**Figure 8** summarizes the chlorine balance observed by the inventors. A closed chlorine balance could be ascertained: **(1)** represents the share of Cl lost on the solid surface; **(2)** HCI; **(3)** recombined Cl₂ which can be recycled; **(4)** selectively formed CH₃Cl. An overall process would thus convert 30 % of the active Cl directly to the valuable product and 60 - 70 % of Cl₂ will have to be redirected into the reactor after product separation.

Two efficient product separation methods from the gas phase have been demonstrated by the inventors (**Figure 5**):
- If pure CH₃Cl product is aimed, the gas stream can be led through a cold trap held at ca. - 30°C allowing the condensation of CH₃Cl (bp. - 25 °C), while dilute amounts of Cl₂ (bp. - 35 °C) in unconverted CH₄ (bp. - 161 °C) and carrier gas N₂, He or Ar (all bp. < - 185 °C) can be recycled to the reactor.
- If a diluted product in a solvent is aimed, the product stream can be directly washed in such a solvent for example MeOH until saturation is reached, while the other gases in the stream are barely soluble in the given solvent and can be recycled into the reactor.

### 1.2.2 Plug flow setup

Homogeneous mixtures of milling medium (ceria, alumina or silica respectively) and TCCA were prepared by gentle grinding and loaded into reactor tubes (length 28 cm, ID 6.5 mm, OD 10 mm, made of steel-1.4571). Temperature was controlled inside the bed with a K-type thermocouple. Continuous sampling of the outlet gas flow was achieved using a direct connection to the same GC-setup described above.

### 1.3. Characterization

FTIR Attenuated total reflection IR spectra were recorded on a Nicolet Magna-IR 560 with a mercury cadmium telluride (MCT) detector cooled with liquid N₂. 32 scans were accumulated and averaged to improve the signal to noise.

Raman measurements were performed using the 532 nm line of a Nd:YAG laser for excitation in the region 50-3500 cm⁻¹ on a Renishaw inVia Qontor confocal Raman microscope equipped with a CCD detector.

NMR spectra were measured using a Bruker AVIII 300 Nanobay spectrometer. Spectra were referenced to residual protons of the deuterated solvent. Chemical shifts are stated in parts per million (ppm) downfield of tetramethylsilane.

N₂-physisorption measurements were carried out on a 3Flex from Micromeritics while samples were degassed on the SmartVacPrep setup from Micromeritics. Samples were degassed first at 90 °C for 1 h, then at 300 °C for 10 h, and measured from 0.01 p/p₀ onwards as no microporosity was expected. Specific surface areas (SSA) were calculated with the Brunauer Emmett Teller (BET) equation using MicroActive software from Micromeritics in the 0.05 - 0.3 p/p₀ range. Total pore volumes were calculated from the volume adsorbed at relative pressures of p/p₀ = 0.99.

DSC All samples were measured on a Mettler Toledo TGA/DSC 1 in 100 µL aluminium crucibles with a heating rate of 5 °C min⁻¹ under constant Ar flow of 60 mL min⁻¹ controlled by GC 200 gas controller.

Particle size measurements have been performed by transmission electron microscopy (TEM) on a Hitachi H7100 at 100 kV acceleration. Samples were placed on copper Lacey grids coated with 3 nm of carbon.

X-ray powder diffraction (XRPD) All samples were measured on STOE Stadi P diffractometers, equipped with a curved Ge (111) monochromator and sealed X-ray tubes. CeO₂ samples were measured with Mο-Kα₁ (λ = 0.70930 A, 50 kV, 40 mA) radiation and a Mythen K1 silicon strip detector, heating experiments were performed with Mο-Kα₁ radiation using a STOE HT1 oven and a linear position sensitive detector (STOE lin PSD, 10% CH₄ in Kr), all other samples with Cu-Kα₁ (λ = 1.54060 Å, 40 kV, 40 mA) radiation and a lin PSD (10% CH₄ in Ar). For structure determination of TCCA and cyanuric acid, the Bragg reflections were indexed with DICVOL yielding reliable unit cell parameters, refined with Pawley methods. The volume increments of Hofmann indicated *Z* = 2, and *Z'* = 1/3 for TCCA and *Z* = 4, and *Z'* = ½ for cyanuric acid (*Z*/*Z*' = number of formula units in the unit cell/asymmetric unit), and due to systematic extinctions the most reliable space group *P*6₃/*m* for TCCA and C2/c for cyanuric acid were determined, due to *m* symmetry of the molecule. The structures were solved in real space with the program DASH using a molecular model. The solved structures were Rietveld refined using program TOPAS. Pseudo atoms on symmetry equivalent positions were used to restrain bond lengths and angles; in addition, the aromatic rings were restrained to be flat. The background was fitted with a polynomial function. For all non-hydrogen atoms the same isotropic Debye-Waller factor *Bᵢₛₒ* was used, while the one for hydrogen atoms was constrained to be 1.2·*Bᵢₛₒ*.

DFT calculations were performed with Spartan software using a MMFF force field and EDF2 level of theory.

X-ray photoelectron spectroscopy (XPS) surface elemental analyses were performed on a SPECS device.

As illustrated above, the inventors have developed a mechanochemical strategy to selectively convert an aliphatic hydrocarbon, such as an alkane having 1 to 8 carbon atoms, preferably having 1 to 6 carbon atoms such as methane, ethane, propane, butane, pentane, hexane and their isomers, into the corresponding haloalkanes, exemplified for methane into chloromethane, at overall benign conditions employing a solid particulate halogen source, for example trichloroisocyanuric acid (TCCA) as a cheap, non-toxic and non-corrosive solid chlorinating agent, optionally in combination with a halogen in gas form, wherein the reaction conditions are chosen to react the alkane in its gaseous phase with the halogen source. TCCA is shown to release active chlorine species upon milling with Lewis acidic materials which allow to functionalize methane at low temperatures (< 150 °C). The used setup allows for the careful screening of catalytically relevant parameters: temperature, contact time and the effect of kinetic energy. A thorough parameter optimization led to a maximum methane chlorination rate of 0.8 µmol(CH_{4, con.}).(g.s)⁻¹. Compared to the thermal reaction of methane with TCCA, the inventors observed that mechanochemical activation permitted significantly lower reaction temperatures (90 vs. 200 °C) at a drastically improved CH₃Cl selectivity (95 % vs 66 % at a conversion level of 30 %). Given the selectivity of close to 100 %, the gravimetric productivity of the herein described process reaches about 150 g CH₃Cl kg⁻¹h⁻¹, in the range of other large scale technical processes for the production of bulk chemicals.

## Claims

1. Process for the direct halogenation of an aliphatic hydrocarbon **characterized by** reacting an aliphatic hydrocarbon having 1 to 8, preferably 1 to 6 carbon atoms, more preferably 1 to 3 carbon atoms, in its gaseous state with a solid particulate halogen source in the presence of a particulate inert milling medium in a reactor, optionally in the presence of milling balls, at a temperature below the decomposition temperature of said solid particulate halogen source wherein mechanical forces are applied to the reaction system, whereby the reactor is equipped with at least one inlet and at least one outlet and the aliphatic hydrocarbon is led into the reactor preferably at a continuous flow rate and the resulting reaction mixture is led out of the reactor.

2. Process according to claim 1, wherein the solid particulate halogen source is selected from halogen containing electrophilic molecules, preferably nitrogen-halogen bond containing molecules being preferably selected from trichloroisocyanuric acid, tribromoisocyanuric acid, N-chlorosuccinimide or N-bromosuccinimide, and optionally in the presence of halogen gas

3. Process according to any claim 1 or 2 wherein the reaction temperature is kept in the range of 75°C to 150°C, more preferably between 110°C and 130°C

4. Process according to any of claims 1 to 3, wherein the particulate inert milling medium is selected from a Lewis acidic material selected from titania, zirconia, γ-alumina, ceria or mixtures thereof.

5. Process according to any of claims 1 to 4, wherein the particle size of the inert particulate milling medium is in a size range of below 500nm, preferably below 100nm and more preferably below 50nm.

6. Process according to any of claims 1 to 5, wherein the milling balls are balls made of steel, WC, Agate, ZrO₂, optionally coated with PTFE or combinations thereof, said balls preferably having a diameter between 0.1 to 10 cm.

7. Process according to any of claims 1 to 6, wherein walls of the reactor are made of or at least partially covered with steel, WC, PTFE, Agate or ZrO₂.

8. Process according to any of claims 1 to 7, wherein the resulting reaction mixture is led out of the reactor and is separated into its components and the obtained unreacted aliphatic hydrocarbon is recycled to the inlet of the reactor.
